Europäisches Patentamt

European Patent Office

Office européen des brevets

(11) Publication number: **0 370 600**
**A1**

## (12) EUROPEAN PATENT APPLICATION

(21) Application number: **89307645.5**

(51) Int. Cl.⁵: **F24J 1/00**

(22) Date of filing: **27.07.89**

(30) Priority: **25.11.88 JP 297536/88**

(43) Date of publication of application:
**30.05.90 Bulletin 90/22**

(84) Designated Contracting States:
**DE FR GB IT**

(71) Applicant: **Seike, Takashi**
**20-26, Kuriki 1-chome Isogo-ku**
**Yokohama-shi Kanagawa-ken(JP)**

(72) Inventor: **Seike, Takashi**
**20-26, Kuriki 1-chome Isogo-ku**
**Yokohama-shi Kanagawa-ken(JP)**

(74) Representative: **Wilkinson, Stephen John et al**
**c/o Stevens, Hewlett & Perkins 5 Quality**
**Court Chancery Lane**
**London WC2A 1HZ(GB)**

(54) **Pyrogenic sheet and method of manufacturing thereof.**

(57) A pyrogenic sheet having a substrate (3) comprising synthetic fibers solely or in admixture with natural fibers and/or regenerated cellulose fibers, with a pyrogenic agent (4) that generates heat in contact with oxygen in air being dispersed and retained therein. The substrate (3) may be sandwitched between other fibrous layers (1,2,12,13,14) for enabling effective and smooth heat-sealing. The pyrogenic sheet of this structure can retain an effective amount of chemical pyrogenic agent with neither displacement nor localization and remain flexible during use.

*F i g. I*

## PYROGENIC SHEET AND METHOD OF MANUFACTURING THEREOF

BACKGROUND OF THE INVENTION

Field of the Invention

The present invention concerns a pyrogenic sheet possessing a particulate chemical pyrogenic agent dispersed and retained therein, as well as a method of manufacturing such a pyrogenic sheet.

Description of the Prior Art

As is well-known, when easily oxidizable material such as powdery pure iron or reduced iron is brought into contact with air in an aqueous medium, preferably, brine under the presence of an adequate catalyst, exothermic catalytic oxidation is taken place to release moderate heat. These oxidizable material, catalyst, etc. are referred to as a chemical pyrogenic (heat-generating) agent and utilized, for example, in so-called disposable chemical pocket warmers.

There have been known pyrogenic sheets that possess an effective amount of chemical pyrogenic agent uniformly dispersed and retained immovably therein with no localization.

For instance, Japanese Patent Publication No. Sho 62-4220 discloses a pyrogenic sheet in which the chemical pyrogenic agent is filled in three-dimensional open-cell material such as polyurethane foams.

However, since the above-mentioned pyrogenic sheet comprises a sheet of open-cell structure as the layer to be filled with the chemical pyrogenic agent, it involves the following problems:

(1) In the case of using iron powder, activated carbon etc. as the chemical pyrogenic agent, a great amount of wood powder or like other natural cellulose powder has to be mixed and filled additionally in the layer as a medium for absorbing, retaining and supplying brine as the aid of the oxidative reaction. Accordingly, it is necessary to increase the thickness or the volume of that layer by so much. Therefore, the size of the pyrogenic sheet is enlarged, as a whole, and its flexibility and the elasticity are reduced remarkably.

(2) In addition, along with the progress of the exothermic reaction during use of the pyrogenic sheet, the iron powder filled therein remarkably swells as it is converted into iron oxide in the course of chemical bonding with oxygen. As a result, it is necessary to use a sheet of large thickness also in view of the expected increase in the volume of the reaction product. Further, since iron oxide forms, together with a great amount of the wood powder, stiff layers along the rigid and strong skeltons of the open cells, the pyrogenic sheet further loses its flexibility and elasticity during use to give undesirable stiff feeling.

SUMMARY OF THE INVENTION

Accordingly, it is a first object of the present invention to provide a pyrogenic sheet which is thin, flexible and highly elastic, as well as a method of easily manufacturing such a sheet.

The second object of the present invention is to provide a pyrogenic sheet capable of containing an effective amount of a chemical pyrogenic agent dispersed and retained therein and preventing leakage of the agent from the sheet as well as a method of easily manufacturing such a sheet.

The foregoing objects of the present invention can be attained, basically, by a pyrogenic sheet including a fibrous substrate made of a non-woven fabric layer solely consisting of or comprising synthetic fibers or a non-woven fibrous layer comprising plant fibers and/or regenerated fibers and synthetic fibers, in which a chemical pyrogenic agent that generates heat in contact with air is dispersed and retained.

In another aspect of the present invention, a pyrogenic sheet includes, a central non-woven fabric layer solely consisiting of or comprising synthetic fibers, intermediate non-woven fabric layers each comprising fusible synthetic fibers and disposed on both outer surfaces of the central non-woven fabric layer and non-woven fibrous top layers each disposed on the outer surface of each of the intermediate non-woven fabric layers.

In a further aspect of the present invention, a pyrogenic sheet includes;
an inner non-woven fabric layer solely consisting of or comprising synthetic fibers and;
outer non-woven fabric layers each comprising fusible fibers and less fusible fibers and disposed on both outer surfaces of the inner non-woven fabric layer.

In a still further aspect of the present invention, all of constituent layers for a pyrogenic sheet are substantially sealed with each other along the periphery threreof.

## DETAILED DESCRIPTION OF THE INVENTION

In one preferred embodiment of the present invention, the central non-woven fabric layer that possesses the pyrogenic agent therein (hereinafter also referred to as the first non-woven fabric layer) may solely consist of synthetic fibers or comprise the synthetic fibers and plant fibers and/or regenerated fibers.

As the material for the synthetic fibers in the first non-woven fabric layer, there can be mentioned polyester, polyethylene, nylon, acryl, polypropylene, polyurethane, acetyl cellulose, vinyl chloride, vinyliden chloride, etc.

It is desirable that the first non-woven fabric at least partially contains fusible synthetic fibers for reliably dispersing and immovably retaining the chemical pyrogenic agent.

As preferred fusible fibers, there can be mentioned, polyester fibers, polyethylene fibers, polypropylene fibers, etc. Fusible synthetic fibers mean here and hereinafer those fibers constituted with the same polymeric material just mentioned above such as polyester, polyethylene, etc. but having lower fusing point as compared with that of other corresponding synthetic fibers used in the pyrogenic sheet. It is preferred to use composite fibers each having a polypropylene core and a fusible polyethylene clad layer.

As the plant fibers which may be used together with the synthetic fibers in the first non-woven fabric layer, there can be mentioned cotton, linnen, pulp, etc. Among them, cotton and pulp are preferred, with absorbent cotton being particularly effective. As the regenerated fibers, there can be mentioned rayon fibers made of viscose rayon and cupra ammonium rayon generally known as bemberg, cupuro, etc. saponified acetate, etc., rayon fibers being preferred. In a case where the first non-woven fabric layer comprises synthetic fibers, plant fibers and/or regenerated fibers, the layer may comprise from 10 to 95 % of the plant fibers and/or regenerated fibers and from 90 to 5 % of synthetic fibers. In view of the performance for retaining the chemical pyrogenic agent, performance for promoting the exothermic reaction, flexibility of the sheet, toughness thereof given by heat treatment, etc., it is preferred that the plant fibers is contained by from 60 to 20 %.

For the chemical pyrogenic agent filled in the central or first non-woven fabric layer, any material that is highly reactive with oxygen in air to produce heat may be used with no particular restrictions. There can include, for example, easily oxidizable material such as pure iron, reduced iron, nickel, sodium sulfide and sodium sulfite, oxidation promoter such as sodium chloride, calcium chloride, magnesium chloride, mineral acid and water, catalyst such as activated carbon, carbon powder and a mixture of copper compound and manganese compound, as well as water retaining material such as perlite, sepiolite, vermiculite, diatomaceous earth, active white clay, silica gel, zeolite and water absorbent resin.

The intermediate non-woven fabric layer containing fusible fibers (hereinafter also referred to as the second non-woven fabric layer) disposed on both surfaces of the first non-woven fabric layer may consist solely of fusible fibers or may comprise plant fibers and/or regenerated fibers and fusible fibers. The plant fibers and the regenerated fibers in the second non-woven fabric layer may properly be selected from those fibers exemplified as the constituent for the first non-woven fabric layer.

The fusible fibers in the second non-woven fabric layer function to intrude between individual fibers of the central non-woven fabric layer and the non-woven fibrous top layer (described more specifically later) disposed respectively on the inner and the outer sides of the second or intermediate non-woven fabric layer upon heat-sealing described later, whereby the respective layers can be bonded with each other.

Use of polypropylene-polyethylene composite fibers as described above as the fusible fibers desirably contributes to the fusion bonding between the non-woven fibrous top layer and the central or first non-woven fabric layer.

The non-woven fibrous top layer disposed as the outermost layer for the pyrogenic sheet according to the present invention is made of plant fibers and/or regenerated fibers. In a general meaning, they include usual non-woven fabrics obtained by dry method, paper or like other products obtained by wet method, filter paper or like other products obtained by semi-wet method, as well as those products obtained by applying further processing to the above mentioned products, paper being particularly preferred.

Since the non-woven fibrous top layer has no fusibility, if the layer is heated under pressure, e.g., by roll pressing, undesirable adherance of the pyrogenic sheet onto the rolls can be avoided.

Further, if cotton, paper or filter paper is used as the non-woven fibrous top layer, the pyrogenic aid such as brine, when scattered to the pyrogenic sheet, can be absorbed to the sheet surface with no repellency.

In addition, since the layer, particularly, paper layer has a fine network structure, leakage of the chemical pyrogenic agent from the pyrogenic sheet can surely be prevented.

In another simplified embodiment of the pyrogenic sheet in the present invention, the sheet may have a three-layered structure only consisting of an inner layer for possessing a pyrogenic agent and made of fibrous material which is substantially the same as that used in the first non-woven fabric layer in the previous embodiment and outer layers made of non-woven fabrics each containing a mixture of fusible fiber and less fusible fibers, with the latter being the main ingredient and, appended respectively to both surfaces of the inner layer.

The fusible fibers and the less fusible fibers used for the outer non-woven fabric layers may properly be selected from those fibers mentioned in the previous embodiment.

In this case, however, since the outer non-woven fabric layer is in direct contact with the rolls upon heating the sheet under pressure, it is desirable that the layer contains the less fusible fibers as the main ingredient, in other words, contains the fusible fibers only in such an amount as required for the subsequent heat sealing applied by means of the roll pressing.

The content of the fusible fibers in the outer non-woven fabric layer may range about from 10 to 95 %, while the content of the less fusible fibers may range about from 90 to 5 %. Desirably, the content of the less fusible fibers is at least 50 % by weight.

In a further simplified embodiment, the pyrogenic sheet in accordance with the present invention, may comprise only one fibrous substrate comprising plant fibers and/or regenerated fibers and synthetic fibers in which the chemical pyrogenic agent is dispersed and retained immovably. Preferred examples of the chemical pyrogenic agent, plant fibers, regenerated fibers and synthetic fibers in this modified pyrogenic sheet are the same as those described previously.

Any woven or non-woven fabric may be used as the fibrous substrate and, a laminated non-woven fabric of appropriate network structure is particularly desirable since leakage of the chemical pyrogenic agent through the bottom face of the fibrous substrate can be avoided. Also in this case, mixing ratio for the respective fibers is from 10 to 95 % of the plant fibers and/or regenerated fibers and from 90 to 5 % of the synthetic fibers, from 60 to 20 % of the plant fibers being more preferred.

The pyrogenic agent can be dispersed and retained in the substrate of the pyrogenic sheet, for example, by scattering particulate chemical pyrogenic agent on the substrate and, if necessary, laying another substrate thereover to constitute a sandwitch structure.

In a case where the substrate has an appropriate thickness, the substrate may be vibrated or shaken after scattering the particulate pyrogenic agent on the substrate, thereby settling the chemical pyrogenic agent through the fibrous substrate. In this case, covering by another substrate may be saved.

If the heat-processing under pressure is applied by means of embossing for the sheet, continuous production is possible at a constant temperature and a constant pressure. In addition, this can also facilitate to retain a great amount of chemical pyrogenic agent to the inside of the fibrous substrate.


DESCRIPTION OF THE ACCOMPANYING DRAWINGS

These and other objects, as well as advantageous features of the present invention will become more apparent by reading the following descriptions referring to the preferred embodiments of the present invention in conjunction with the appended drawings, wherein

Figs 1(a) - (i) are, respectively, charts for successive steps of one embodiment for the method of manufacturing a pyrogenic sheet according to the present invention;

Figs 2(a) - (c) are charts for a portion of steps for another method of manufacturing a pyrogenic sheet according to the present invention;

Figs 3(a),(b) are charts for a portion of steps for a further method of manufacturing a pyrogenic sheet according to the present invention and

Fig. 4 is a longitudinal cross sectional view illustrating the structure of the pyrogenic sheet according to the present invention.


Preparation of the Pyrogenic Sheet According to the Present Invention

The method of manufacturing the pyrogenic sheet according to the present invention is to be explained referring to the drawings.

4

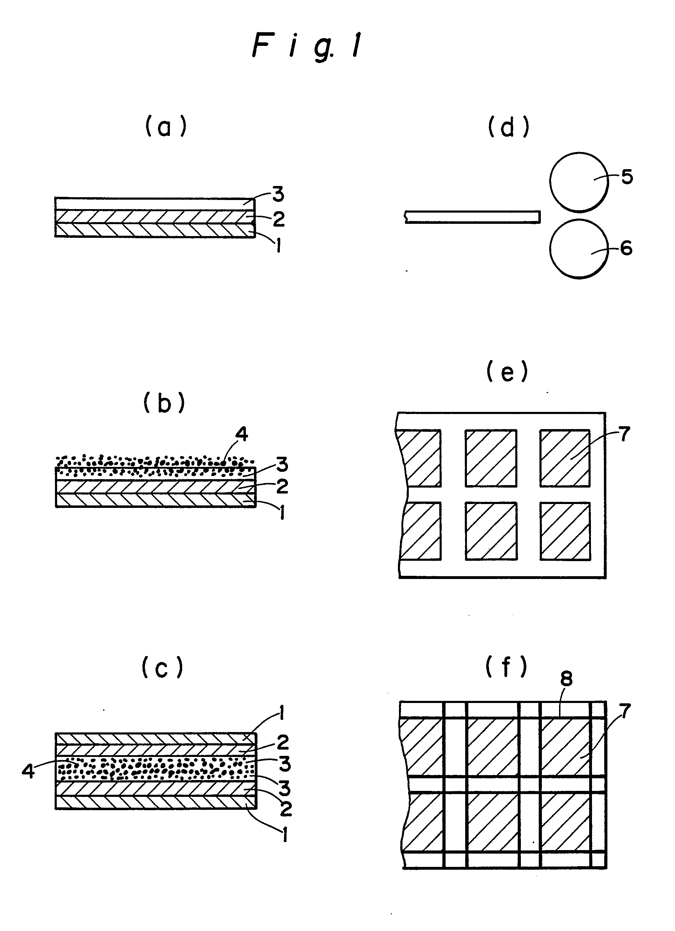

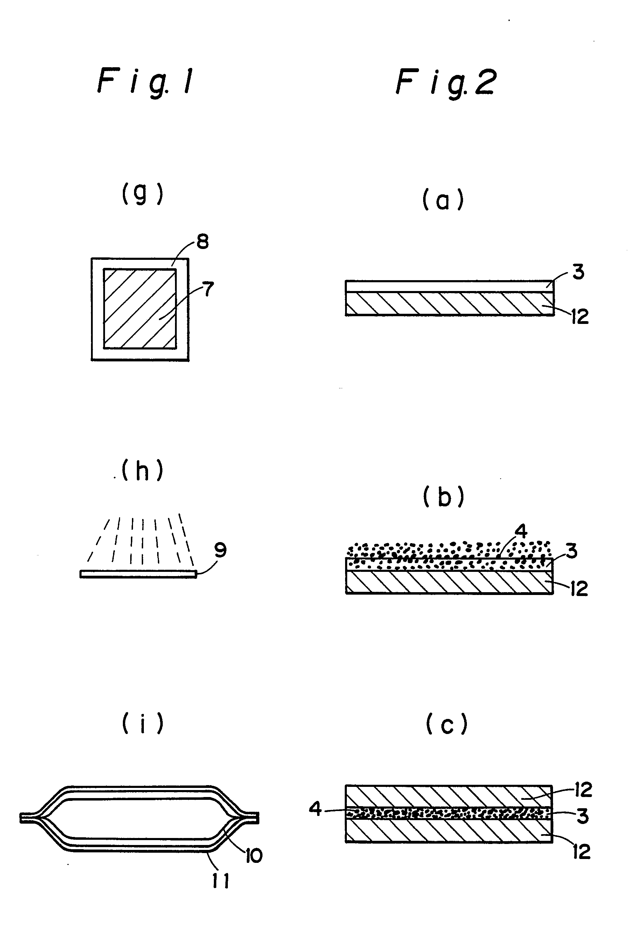

Figs. 1(a) through (i) are, respectively, step charts illustrating one embodiment for the method of manufacturing the pyrogenic sheet according to the present invention.

At first, as shown in Fig. 1(a), non-woven fibrous top layer 1, second non-woven fabric layer (intermediate layer) 2 and first non-woven fabric layer (central layer) 3 are successively stacked upwardly in this order. Then, particles 4 of a chemical pyrogenic agent, for example, iron powder, activated carbon, etc. are scattered onto the surface of the first non-woven fabric layer 3, divisionally, at each of a plurality of square-like regions. Then, the particles 4 are filled into the gap between each of individual fibers in the first non-woven fabric layer 3. The shape of the region for scattering the chemical pyrogenic agent is not restricted only to the illustrated square shape but it may be of any other optional shapes such as of circular, elliptic, etc.

Then, as shown in Fig. 1 (c), another second non-woven fabric layer 2 and another non-woven fibrous top layer 1 are successively laminated on the surface of the scattered particles 4 and then they are heated under pressure between an emboss roll 5 and a plain roll 6 shown in Fig. 1(d).

Upon heat-pressing operation by the rolls 5, 6, the particles 4 are firmly set to the synthetic fibers contained in the first non-woven fabric layer 3 by the fusion of the fibers and, at the same time, partially enclosed individually between each of the fibers, by which the particles 4 can surely be retained immovably in the first non-woven fabric layer 3.

In addition, since the second non-woven fabric layer 2 contains the fusible fibers, the fusible fibers are bonded with the synthetic fibers in the first non-woven fabric layer 3 by means of fusion at a number of positions along the plane where the particles 4 are dispersed, thereby preventing the first non-woven fabric layer 3 and the second non-woven fabric layer 2 from peeling to each other.

In addition, since the fusible fibers in the second non-woven fabric layer 3 are fused and penetrate into the gaps between each of the indivisual fibers in the non-woven fibrous top layer 1, peeling between the second non-woven fabric layer 2 and the non-woven fibrous top layer 1 can also be prevented.

The heat-pressing operation by the rolls 5, 6 shown in Fig. 1(d) is applied under such conditions that the particles 4 can surely be retained in the first non-woven fabric layer 3, peeling between each of the non-woven fabric layer layers can be prevented, as well as the flexibility of the sheet can be maintained.

The conditions for the heat-pressing are desirably set such that the temperature is from 120 to 200° C and the pressure is from 5 to 20 kg/cm², although they somewhat vary depending on the kind of layers for each of the layerous non-woven fabric layers 1, 2 and 3, thickness for each of them, etc.

Fig. 1(e) schematically shows the state of the sheet as viewed from above, in which 7 denotes a region for filling the particles 4 of the chemical pyrogenic agent.

Then, after applying heat-seal embossing such that only the regions 8 represented by fat solid lines are sealed as shown in Fig. 1(f), the sheet is divisionally cut along each of the heat-sealed portions. Thus, individual pyrogenic sheets 9 each comprising the region 7 containing the particles 4 dispersed and retained therein and sealed portion 8 formed at the periphery thereof can be obtained as shown in Fig. 1(g).

The heat sealing is applied for preventing the particles 4, which may be present at the boundary between the non-woven fabric layer 2 and 3, from leaking at the periphery of the sheet. Desirable conditions for heat-pressing by the emboss roll are selected as from 120 to 250° C of temperature and from 5 to 30 kg/cm² of pressure. In addition to the emboss rolling, the heat sealing may also be applied by other methods such as inpulse heat sealing.

During embossing fabrication shown in Figs 1(d) and (f), since the infusible non-woven fibrous top layer 1 such as made of paper is in contact with the roll surface, there is no undesirable heat adherance of the non-woven fibrous top layer 1 to the roll surface. This can improve the yield of the pyrogenic sheet, as well as the operationability of the embossing fabrication.

Subsequently, brine as the pyrogenic aid is sprayed on the thus obtained pyrogenic sheet 9 as shown in Fig. 1(h). In this case, since the non-woven fibrous top layer 1 such as made of paper is highly water absorptive, brine sprayed on the surface of the non-woven fibrous layer 1 is uniformly penetrated over the entire area of the sheet without repellation. The penetrated brine is uniformly retained in the second non-woven fabric layer 2 and then further penetrates into the first non-woven fabric layer 3.

Then, the pyrogenic sheet 9 thus sprayed with brine is enhoused, as shown in Fig. 1(i), to the inside of an inner bag comprising a film 10 (for example, a film made of polyethylene or ethylene-vinyl acetate copolymer formed with a plurality of perforations) at the inside and a surface layer 11 (for example, various kinds of non-woven fabric layers selected depending on the application use) at the outside thereof for controlling air permeability. The bag has an opening at one side and is heat-sealed along other sides. The non-woven surface layer 11 may be saved depending on the application use. After enhousing the pyrogenic sheet, the inner bag is heat-sealed at its opening and is air-tightly sealed in another bag comprising an oxygen-impermeable film not illustrated.

In another embodiment of the present invention, a pyrogenic sheet can also be prepared, as shown in Fig. 2(a), by laying the inner non-woven fabric layer 3 (corresponding to the first non-woven fibric layer in the previous embodiment) over an outer non-woven fabric layer 12 (corresponding to the second non-woven fabric layer in the previous embodiment), scattering particulate chemical pyrogenic agent 4 thereover as shown in Fig. 2(b), further laying another outer non-woven fabric layer 12 thereover as shown in Fig. 2(c) and then applying the same procedures as those shown in Figs 1(d) through (i) previously described.

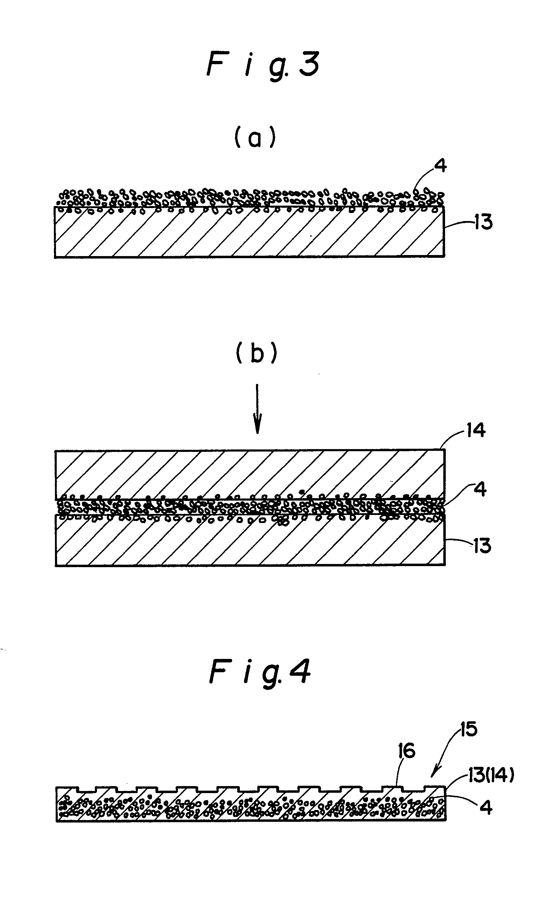

Figs 3(a), (b), respectively, show step charts illustrating a further embodiment of manufacturing a pyrogenic sheet in accordance with the present invention.

At first, as shown in Fig. 3(a), particulate chemical pyrogenic agent 4 is uniformly scattered on a fibrous substrate 13 comprising plant fibers and/or regenerated fibers and synthetic fibers. Then, as shown in Fig. 3(b), another fibrous substrate 14 comprising plant fibers and/or regenerated fibers and synthetic fibers are laid thereover.

The entire laminate comprising the fibrous substrate 13, the chemical pyrogenic agent 4 and the fibrous substrate 14 are embossed along both of upper and lower sides under heating. In this case, heating may be applied only from one side or from both sides of the fibrous substrates 13 and 14. In a case where heating is applied only on one side, the substrate 14 may be saved and, instead, a thin film or sheet can be appended on the other side for preventing the leakage of the chemical pyrogenic agent. Fig. 4 shows a pyrogenic sheet 15 prepared by embossing, in which an embossed layer is formed on the upper surface. Then, peripheral embossing is further applied as shown by 8 in Fig. 1(f).

The present invention, having been constituted as described above, can provide the following advantageous merits.

(1) Since the chemical pyrogenic agent is dispersed and retained in the fibrous layer, an effective amount of the chemical pyrogenic agent can be held between individual fibers with neither displacement nor localization. In particular, if the fibrous substrate is made of non-woven fabric layer, the chemical pyrogenic agent can surely be retained. In addition, if the substrate is made of non-woven fabric layer or plant fibers and/or regenerated fibers, the sheet can be made highly flexible.

(2) In a case where the intermediate non-woven fabric layer contains fusible fibers, since the fusible fibers are melted and partially fused with or intrude into the adjacent central layer and/or top layer, upon heating of the sheet under pressure, it is possible to prevent peeling between the sheets-constituent layers while maintaining the flexibility of the pyrogenic sheet.

(3) Upon heat-sealing the peripheral portion of the pyrogenic sheet, since the fusible fibers contained in the sheet constituent layer are melted and fused with and/or penetrate into the adjacent layers, heat-sealing can be conducted easily and completely.

Accordingly, even if the heat-sealed portions are cut at any portion, the chemical pyrogenic agent does not leak from the cut face. As a result, it is possible to mass-produce pyrogenic sheets by, at first, preparing a wide and long original stock sheet for the pyrogenic sheet, and then cutting it into individual sheets each of a desired shape and size.

(4) Even if the volume of the pyrogenic agent is increased by the conversion into oxides along with the progress of the oxidative reaction, expansible structure of the flexible and resilient non-woven fabric layer can afford such increased volume, and it is no more necessary to use a sheet of unnecessarily large thickness.

(5) Since brine, etc. scattered as the pyrogenic aid on the sheet surface uniformly penetrates the non-woven fibrous top layer and is retained in the second and the first non-woven fabric layers, brine can be supplied effectively to the chemical pyrogenic agent.

In addition, when the fibrous substrate comprising plant fibers and/or regenerated fibers together with synthetic fibers is used, since these fibers can absorb, retain and supply brine by themselves, it is no more necessary to incorporate a great amount of wood powder, etc. as water-retainer.

(6) Since the chemical pyrogenic agent can be retained immovably over the entire portion uniformly with no localization, performance of the products using the pyrogenic sheet is improved and the application use for the pyrogenic sheet can be extended.

Specifically, in a case of using the pyrogenic sheet for so-called chemical pocket warmers, since the chemical pyrogenic agent neither lumps nor causes localized generation of heat, a warmer of reduced thickness, comfortable to use and causing less risk of burnt can be obtained. In addition, when the sheet is used as an insole for shoes, the entire surface can be warmed uniformly and the heat can be generated continuously, while maintaining the resiliency of the sheet. In addition, since there is no unevenness, comfortable feeling can be obtained upon wearing shoes.

In a case of using the pyrogenic sheet as warming means used for lumbago, stiff shoulders, neuraligia or habitual chills, it is possible to optionally select appended area, shape, temperature and heat-keeping period depending on the places where the sheet is applied. In addition, therapeutical effects can also be improved by the combined use with hot packs, medicines, etc.

EXAMPLES

The present invention is to be described more specifically referring to examples and comparative examples, but it should be noted that the present invention is not restricted only to such examples.

Example 1

Tissue paper as non-woven fibrous layer (top layer) 1 (23 g/m$^2$ weight, 60 um thickness), second non-woven fabric layer (intermediate layer) 2 comprising 60 % absorbent cotton and 40 % fusible polyester fibers (80 g/m$^2$ weight, 3 mm thickness) and first non-woven fabric layer (central layer) 3 comprising 50 % polyester and 50 % fusible polyester fibers (40 g/m$^2$ weight, 3 mm thickness) were successively stacked into a three-layered substrate. Then, particulate chemical pyrogenic agent prepared by mixing 100 parts by weight of iron powder (70 um in average particle size) and 10 parts by weight of activated carbon (24 um in average particle size) was uniformly scattered in an amount of 12 g within a area of 13 cm x 9 cm size and then applied with embossing. In this case, embossing was conducted at a temperature of 160 °C for both of the upper and the lower rolls and under a pressure of 10 kg/cm$^2$. An aqueous 20 % brine was sprayed by 5.4 g as a pyrogenic aid to the thus finished pyrogenic sheet (2 mm thickness), which was then sealed in an inner bag. The inner bag was prepared from a punctured polyethylene film (30 um) and a non-woven fabric layer comprising 50 % polyester and 50 % absorbent cotton (80 g/m$^2$ weight, 0.6 mm thickness) by applying heat-sealing along four sides (refer to Fig. 1(i)).

Then, the inner bag was tightly sealed in another bag made of an air impermeable film. When the pyrogenic sheet thus controlled with the air permeability was measured for the pyrogenic performance in accordance with JIS 4100, 1985 (for disposable pocket warmer), the following results were obtained.

| Maximum temperature | 55 °C |
|---|---|
| Temperature-keeping time | 6 hours |
| Heat-continuing time | 7 hours |

Warming effect was observed uniformly over the entire surface. The air permeability of the punctured polyethylene film was 0.6 cc/cm$^2$. sec (measured by Fragil permeability tester).

Example 2

A pyrogenic sheet was manufactured under the same conditions as those in Example 1 except for using two non-woven fabric layers each comprising 50 % polyester and 50 % fusible polyester fibers stacked to each other as the first non-woven fabric layer 3 and spraying 32 g of the pyrogenic agent and 12 g of 20 % brine, to obtain a pyrogenic sheet of 3 mm thickness. The pyrogenic performance was as shown below.

| Maximum temperature | 60 °C |
|---|---|
| Temperature-keeping time | 16 hours |
| Heat-continuing time | 17 hours |

Example 3

A pyrogenic sheet was manufactured under the same conditions as those in Example 1 except for controlling the air permeability of the punctured film to 3 cc/cm$^2$.sec and spraying 3.2 g of 20 % brine. The pyrogenic performance was as described below.

| Maximum temperature | 76° C |
|---|---|
| Temperature-keeping time | 1.5 hours |
| Heat-continuing time | 2 hours |

Example 4

Embossing was applied for the pyrogenic sheet in the same manner as in Example 1 except for using non-woven fabric layer comprising 100 % absorbent cotton (120 g/cm$^2$ weight, 0.5 mm thickness) as the non-woven fibrous top layer 1, non-woven fabric layer comprising 100 % ES fibers (having polypropylene core and polyethylene clad) (20 g/m$^2$ weight, 200 um thickness) as the second non-woven fabric layer 2, and non-woven fabric layer comprising 50 % polyester and 50 % fusible polyester fibers as the first non-woven fabric layer 3 and previously bonding the first non-woven fabric layer 3 with the second non-woven fabric layer 2 by means of adhesives.

12 g of the pyrogenic agent was scattered and embossing was applied at a temperature of 180° C for both of the upper and the lower rolls at a pressure of 10 kg/cm$^2$ to obtain a pyrogenic sheet of 2.5 mm thickness. Then, 3,3 g of 20 % brine was sprayed. Air permeability of the polymer film was controlled to 3 cc/cm$^2$. sec and the pyrogenic performance was measured. The results are as shown below.

| Maximum temperature | 80° C |
|---|---|
| Temperature-keeping time | 1 hour |
| Heat generation-continuing time | 1.5 hour |

Example 5

A pyrogenic sheet was manufactured under the same conditions as those in Example 4 except for using two sheets of non-woven fabric layers each comprising 50 % polyester and 50 % fusible polyester stacked to each other as the first non-woven fabric layer 3, scattering 32 of the pyrogenic agent, spraying 12 g of 20 % brine, setting the thickness of the pyrogenic sheet to 3.5 mm and using a punctured polyethylene film (permeability: 0.6 cc/cm$^2$.sec) for the inner bag. The pyrogenic performance is as shown below.

| Maximum temperature | 62° C |
|---|---|
| Temperature-keeping time | 15 hours |
| Heat generation continuing time | 16 hours |

Example 6

A pyrogenic sheet was manufactured in the same procedures as those in Example 1 except for using non-woven fabric layer comprising 50 % polyester and 50 % heat- fusible polyester fibers (40 g/m$^2$, 3 mm thickness) as the first non-woven fabric layer 3, a non-woven fabric layer comprising 60 % cotton, 30 % rayon and 10 % fusible polyester fibers (85 g/m$^2$ weight, 3 mm thickness) as the third non-woven fabric layer 12 shown in Fig. 2, setting the temperature to 200° C both for the upper and the lower rolls and the pressure to 10 kg/cm$^2$ upon embossing. When the pyrogenic performance was measured, the following results were obtained.

8

| Maximum temperature | 55°C |
|---|---|
| Temperature-keeping time | 6 hours |
| Heat generation continuing time | 7 hours |

Comparative Example 1

A pyrogenic sheet was manufactured in the same procedures as those in Example 1 except for using first non-woven fabric layer 3 comprising 50 % polyester and 50 % fusible polyester (40 g/cm² weight, 3 mm thickness) and the second non-woven fabric layer 2 comprising 100 % ES (20 g/m² weight, 200 um thickness), without using non-woven fibrous layer 1. Then, particulate pyrogenic agent was uniformly scattered in an amount of 12 g over an area of 13 cm x 9 cm and then applied with embossing.

Embossing was applied at a temperature of 150°C both for the upper and the lower rolls at a pressure of 10 kg/cm². However, since the ES non-woven fabric layer clung to the rolls upon embossing fabrication, the temperature was lowered to 120°C, by which a sheet-like product could be obtained, but with insufficient bonding between the first non-woven fabric layer 3 and the second non-woven fabric layer 2. When 5.4 g of 20 % brine was sprayed as the pyrogenic aid to the thus resultant pyrogenic sheet (2 mm thickness), the brine formed droplets at the surface and hardly penetrated as far as the pyrogenic agent. Further, the pyrogenic powder was leaked considerably from the surface of the pyrogenic sheet.

Example 7

Particulate chemical pyrogenic agent 4 comprising 100 parts by weight of iron powder, 10 parts by weight of activated carbon and 5 parts by weight of water absorbent resin in admixture was uniformly scattered at 1700 g/m² on a fibrous substrate 13 comprising non-woven fabric layer containing 60 % absorbent cotton and 40 % fusible polyester fibers (73 g/m² weight, 4 mm thickness) (refer to Fig. 3(a)). Then, a fibrous substrate 14 comprising the non-woven fabric layer of the identical composition with that of the substrate 13 was covered thereover (refer to Fig. 13(b)) and the entire portion of the substrate 13, chemical pyrogenic agent 4 and another substrate 14 was heated under pressure by means of embossing. The embossing was carried out by using an emboss roll heated to 160°C as the upper roll and a plain steel roll heated to 160°C as the lower roll under a pressure of 8 kg/cm².

When 20 % brine was sprayed at 550 g/m² as the pyrogenic aid to the thus resultant pyrogenic sheet (2 mm thickness), moderate heat generation was taken place. The pyrogenic sheet was cut into 150 mm length and 100 mm width, placed in a packaging bag comprising non-woven fabric layer, etc. having a controlled air permeability and the pyrogenic performance was measured in accordance with JIS S 4100 - 1985 (for disposal pocket warmer). The maximum temperature was 50°C, the average temperature was 48°C, the heat continuing time was 13 hours. Thus, moderate and stable warming effect was observed. Further, the sheet had moderate flexibility and appropriate rigidity, as well as showed satisfactory feeling upon putting. Further, when the sheet was placed in an airtight bag to be free from contact with oxygen, the exothermic reaction was terminated to enable long time storage. When the sheet was taken out from the air tight bag, heat generation was initiated again.

Example 8

A pyrogenic sheet (2 mm thickness) was manufactured in the same procedures as those in Example 7 except for using a non-woven fabric layer comprising 10 % absorbent cotton, 50 % rayon and 40 % fusible polyester fibers (60 g/m² weight, 3 mm thickness) for each of the fibrous substrate 13, 14.

20 % brine was sprayed at 370 g/m² to the pyrogenic sheet and the pyrogenic performance was measured in the same manner as in Example 7. Maximum temperature was 50°C, the average temperature was 48°C and, the heat continuing time was 7.5 hours. Thus, moderate and stable warming effect was observed. Further, the sheet had an appropriate flexibility and an adequate toughness and also provided satisfactory feeling upon putting.

Example 9

A pyrogenic sheet (1.5 mm thickness) was manufactured in the same manner as in Example 7 except for using a non-woven fabric layer comprising 50 % absorbent cotton, 10 % rayon and 40 % fusible polyester fibers (60 g/m² weight, 3 mm thickness) and scattering the chemical pyrogenic agent 4 at 1070 g/m².

20 % brine was sprayed at 355 g/m² to the pyrogenic sheet to measure the pyrogenic performance in the same manner as in Example 7. The maximum temperature was 49°C and average temperature was 45°C and, the heat continuing time was 6.7 hours. Thus soft and stable warming effect was observed. Further, the sheet had an appropriate softness and adequate rigidity and could provide satisfactory feeling upon putting.

Example 10

On a non-woven fabric layer comprising 89 % linter pulp and 11 % polyethylene fibers (100 g/m² weight, 1 mm thickness) as the outer layer, another non-woven fabric layer of composition identical with and coarser and thicker than the outer layer (100 g/m² average weight, 5 mm thickness) was placed as an inner layer. Then, the chemical pyrogenic agent used in Example 7 was scattered at 1500 g/m² thereover and the another outer non-woven fabric layer was laid further thereover and then a pyrogenic sheet (3 mm thickness) was manufactured in the same procedures as in Example 7.

To the pyrogenic sheet, 20 % brine was sprayed at 540 g/m² and the pyrogenic performance was measured in the same manner as in Example 7. The maximum temperature was 45°C, the average temperature was 44°C and the heat-continuing time was 11 hours. Thus, moderate and stable warming effect was observed. Further, the sheet had an appropriate flexibility and moderate rigidity and also provided satisfactory feeling upon putting.

Comparative Example 2

A pyrogenic sheet was manufactured in the same manner as in Example 7 except for using a non-woven fabric layer comprising polyester fibers and polyethylene fibers (50 g/m² weight, 0.26 mm thickness) as a fibrous substrate. The pyrogenic sheet could not retain the chemical pyrogenic agent sufficiently. Further, since the substrate was fused to the roll during embossing, the fabrication was difficult.

Then, another pyrogenic sheet (0.5 mm) was manufactured by lowering the temperature to 120°C both for the upper and the lower rolls and reducing the scattering amount of the chemical pyrogenic agent to 400 g/m². Although the pyrogenic sheet could retain the chemical pyrogenic agent, no heat generation occurred.

Comparative Example 3

A non-woven fabric layer comprising 8 % rayon and 92 % polyester fibers bonded with acrylic adhesives (100 g/m² weight, 3 mm thickness) was placed on an adhesive non-woven fabric layer prepared by applying an ethylene-vinyl acetate copolymer adhesion layer to a non-woven fabric layer comprising 5 % rayon, 25 % polyester fiber and 70 % nylon (60 g/m² weight, 0.3 mm thickness). Then, the chemical pyrogenic agent used in Example 7 was scattered at 1700 g/m² and, further thereover, another adhesive non-woven fabric layer as described above was covered. Subsequently, the pyrogenic sheet (2.5 mm thickness) was manufactured in the same procedures as those in Example 7. This pyrogenic sheet showed only poor heat generation perhaps because it could not retain an effective amount of water since the ratio of the synthetic fibers in the pyrogenic sheet was too much.

Comparative Example 4

A pyrogenic sheet (4 mm thickness) was manufactured in the same manner as in Example 7 except for using a non-woven fabric layer comprising 100 % absorbent cotton as the fibrous substrate (130 g/m² weight, 8 mm thickness). Although the heat generation was smooth and the amount of the heat generated was also sufficient in the pyrogenic sheet of this embodiment, the substrate was easily peeled. In addition, since the three-dimensional network structure of the fibers in the fibrous substrate was coarse, the scattered chemical pyrogenic agent was leaked passing through the substrate.

In view of the above, the entire portion were sandwitched at both of upper and lower sides thereof with the adhesive non-woven fabric layers as used in Comparative Example 3 and a pyrogenic sheet was manufactured in the same manner as in Example 7. In this pyrogenic sheet, although the leakage of the chemical pyrogenic agent could be prevented, peeling of the substrate could not be prevented.

## Claims

1. A pyrogenic sheet comprising:
a first non-woven fabric layer solely consisting of or comprising synthetic fibers with a chemical pyrogenic agent being dispersed and retained therein;
second non-woven fabric layers each comprising fusible fibers having a fusing temperature lower than that of said synthetic fibers and appended on both surfaces of said first non-woven fabric layer; and
infusible non-woven fibrous layers each appended on both outer surfaces of said second non-woven fabric layers, in which
respective layers are substantially sealed along the peripheral portion thereof.

2. A pyrogenic sheet as defined in claim 1, wherein the first non-woven fabric layer comprises the synthetic fibers and fusible fibers.

3. A pyrogenic sheet as defined in claim 1, wherein the first non-woven fabric layer comprises the synthetic fibers and plant fibers and/or regenerated fibers.

4. A pyrogenic sheet as defined in claim 1, wherein the second non-woven fabric layer comprises plant fibers and/or regenerated fibers and fusible fibers.

5. A pyrogenic sheet as defined in claim 4, wherein the plant fibers are cotton.

6. A pyrogenic sheet as defined in claim 1, wherein the second non-woven fabric layer comprises fusible fibers.

7. A pyrogenic sheet as defined in claim 1, wherein the infusible non-woven fibrous layer comprises natural fiber or paper.

8. A pyrogenic sheet comprising:
a first non-woven fabric layer solely consisting of or comprising synthetic fibers, with a chemical pyrogenic agent being dispersed and retained therein;
a second non-woven fabric layers each containing fusible fibers having a fusing temperature lower than that of said synthetic fibers and appended on both surfaces of said first non-woven fabric layer, in which
respective layers are substantially sealed along the peripheral portion thereof.

9. A method of manufacturing a pyrogenic sheet, which comprises:
stacking an infusible non-woven fibrous top layer, an intermediate second non-woven fabric layer comprising fusible fibers and a central non-woven fabric layer consisting solely of or comprising synthetic fibers successively in this order,
scattering a chemical pyrogenic agent onto the surface of said central non-woven fabric layer,
stacking another intermediate non-woven fabric layer and another infusible non-woven fibrous top layer successively onto the surface where the pyrogenic agent is scattered and then
heating the layers under pressure thereby sealing predetermined regions thereof.

10. A method of manufacturing a pyrogenic sheet, which comprises:
stacking an outer non-woven fabric layer comprising fusible fibers and less fusible fibers and an inner non-woven fabric layer solely consisting of or comprising synthetic fibers successively,
scattering a chemical pyrogenic agent to the surface of said non-woven fabric, layer then stacking another outer non-woven fabric layer comprising fusible fibers and less fusible fibers over the surface where the pyrogenic agent is scattered and then
heating the layers under pressure, thereby sealing predetermined regions thereof.

11. A pyrogenic sheet, wherein a chemical pyrogenic agent is dispersed and retained in a fibrous substrate comprising plant fiber and/or regenerated fibers and synthetic fibers, and said substrate is applied with heat-treatment under pressure to at least on one surface thereof and applied with substantial sealing along the peripheral portions thereof.

12. A pyrogenic sheet as defined in claim 11, wherein the plant fibers are selected from the group consisting of cotton and pulp and the regenerated fibers are rayon.

13. A pyrogenic sheet as defined in claim 11, wherein the substrate comprises from 10 to 95 % of plant fibers and/or regenerated fibers and from 90 to 5 % of synthetic fibers.

14. A method of manufacturing a pyrogenic sheet which comprises filling a chemical pyrogenic agent in a fibrous substrate comprising plant fibers and/or regenerated fibers and synthetic fibers and then applying heating under pressure to said substrate.

15. A method of manufacturing a pyrogenic sheet as defined in claim 14, which comprises scattering a chemical pyrogenic agent on a fibrous substrate comprising plant fibers and/or regenerated fibers and synthetic fibers, covering another fibrous substrate comprising plant fibers and/or regenerated fibers and synthetic fibers further thereover and then applying heating to the entire portion under pressure.

16. A method of manufacturing a pyrogenic sheet as defined in claim 15, wherein the method of applying heating under pressure is embossing fabrication.

# F i g. 1

Fig.1

(g)

(h)

(i)

Fig.2

(a)

(b)

(c)

# F i g. 3

## ( a )

## ( b )

# F i g. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | JP-A-59 104 933 (MITSUBISHI GAS CHEM. K.K.)<br>* The whole document * | 1,8-10 | F 24 J 1/00 |
| A | PATENT ABSTRACTS OF JAPAN, vol. 7, no. 175 (C179)[1320], 3rd August 1983; & JP-A-58 080 372 (TEIJIN K.K.) 14-05-83 | 1,8-10 | |
| A | EP-A-0 045 642 (MITSUBISHI GAS CHEMICAL CO. INC.)<br>* Page 4, line 5 - page 6, line 22; page 7, line 15 - page 8, line 8; page 8, lines 21-24; page 9, lines 9-18; figure 2 * | 1,8-10 | |
| A | US-A-4 282 005 (SATO et al.)<br>* Column 3, lines 26-47; figure * | 1,8-10 | |
| A | US-E- 32 026 (YAMASHITA et al.)<br>* Column 3, line 64 - column 4, line 40; column 6, lines 29-66; column 8, lines 30-45; figures 1,4,7 * | 1,8-10 | TECHNICAL FIELDS SEARCHED (Int. Cl.5) |
| A | US-A-3 261 347 (SHERMAN)<br>* Column 3, lines 11-54; figure 1 * | 1,8-10 | F 24 J<br>A 61 F<br>A 43 B |
| A | US-A-3 301 250 (GLASSER)<br>* Column 2, line 57 - column 3, line 75; figures 3,4,6 * | 1,8-10 | |
| A | CH-A- 203 260 (DREIDING)<br>* The whole document * | 1,8-10 | |
| P,A | PATENT ABSTRACTS OF JAPAN, vol. 13, no. 328 (C-621)[3676], 24th July 1989; & JP-A-11 04 760 (ASAHI CHEM. IND. CO. LTD.) 25-04-1989 | 1,8-10 | |

The present search report has been drawn up for all claims

| Place of search | Date of completion of the search | Examiner |
|---|---|---|
| THE HAGUE | 01-03-1990 | BELTZUNG F.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)